# FASCICULE DE BREVET EUROPEEN

(11) **EP 1 133 960 B1**
(45) Date de publication et mention de la délivrance du brevet: **11.04.2007**
(21) Numéro de dépôt: 01420046.3
(22) Date de dépôt: 21.02.2001
(51) Int. Cl.: A61F 5/055

(54) **Collier cervical à maintien réglable**
Verstellbare Cervicalstütze
Adjustable cervical collar

(30) Priorité: 13.03.2000 FR 0003418
(43) Date de publication de la demande: 19.09.2001
(73) Titulaire: RICHARD FRERES S.A., 42560 Saint Genest Lerpt (FR)
(72) Inventeur: Richard, Dominique, 43110 Aurec sur Loire (FR)
(74) Mandataire: Maureau, Philippe

(56) Documents cités:
- FR-A- 2 704 424
- US-A- 5 180 361
- US-A- 5 403 266
- US-A- 5 728 054

## Description

L'invention est relative à un collier cervical composé d'un collier, dont les extrémités sont munies de moyens d'accrochage complémentaires permettant son maintien autour du cou, de moyens de renforcement de sa partie antérieure et de moyens de soutien du menton.

Pour le traitement des pathologies des vertèbres cervicales, il est connu d'avoir recours à des colliers cervicaux associés à un soutien mentonnier. Lors du traitement, en particulier lorsque les muscles des vertèbres cervicales se sont raffermis, la mentonnière peut être supprimée pour améliorer le confort du patient. Or, bien souvent cette mentonnière est solidaire du collier, de sorte qu'il faut remplacer l'ensemble du collier par un collier sans mentonnière. Cette façon de procéder augmente les coûts du traitement et, bien souvent, amène le patient à retirer précocement le collier en facilitant les risques de rechute.

Le document US-5 403 266 décrit un collier cervical avec un bourrelet supérieur, en deux tronçons latéraux pour laisser passer la pièce de soutien mentonnier, et un bourrelet inférieur continu et gonflable. Le collier est formé par assemblage de pièces en matériau semi-rigide et ne comprend donc pas de moyens de renforcement.

En raison de sa structure, ce collier ne peut assurer qu'une fonction de maintien rigide et doit être remplacé par un autre, sans mentionnière, lors de l'amélioration de l'état du porteur.

Le document US 5 180 361 montre un collier cervical composé de deux éléments semi rigides, respectivement avant et arrière ; l'élément avant possède un support de mâchoire.

La présente invention a pour objet de remédier à ces inconvénients en fournissant un collier dont les conditions de maintien peuvent être adaptées au fur et à mesure de l'amélioration de l'état du patient, sans qu'il y ait besoin de se procurer un autre collier.

A cet effet, le collier selon l'invention présente les caractéristiques de la revendication 1.

Ainsi, au fur et à mesure du raffermissement des muscles des vertèbres cervicales, il peut être procédé, d'abord, à l'enlèvement de la pièce de soutien mentonnier, puis ensuite à l'enlèvement de la barrette de renfort, et cela sans qu'il y ait besoin de se procurer un autre collier.

Il apparaît donc que le collier selon l'invention permet non seulement de réduire les coûts de traitement, par réduction du nombre de colliers devant être achetés, mais aussi de supprimer les délais d'accoutumance avec chaque nouveau collier, donc d'améliorer le confort du patient.

Dans une forme d'exécution, les moyens de fixation des deux branches de la pièce de soutien mentonnier sur la barrette de renfort comprennent des rivets encliquetables traversant les perforations ménagées dans ces deux pièces.

Dans une autre forme d'exécution de l'invention, les moyens de fixation des deux branches de la pièce de soutien mentonnier sur la barrette de renfort sont montés coulissants avec possibilité de blocage dans deux lumières verticales de réglage, réalisées dans la barrette de renfort.

Ces deux formes d'exécution permettent d'adapter la position de la mentonnière par rapport au collier en fonction des mensurations du patient ou du soutien exigé par le traitement, sans pour autant s'opposer à l'amovibilité de cette pièce de soutien mentonnier, lorsque son usage n'est plus indispensable.

D'autres caractéristiques et avantages ressortiront de la description qui suit en référence au dessin schématique annexé représentant plusieurs formes d'exécution du collier selon l'invention.
Figure 1 est une vue de face en élévation d'une première forme d'exécution du collier, lorsqu'il est posé à plat,
Figure 2 est une vue, en vue du dessus d'une forme d'exécution de la pièce de soutien mentonnier,
Figures 3 et 4 sont des vues en coupe, respectivement III-III et IV-IV de figure 1,
Figures 5 à 7 sont des vues de côté en élévation montrant les différentes configurations pouvant être données au collier selon l'invention,
Figure 8 est une vue partielle de côté en élévation, similaire à la figure 5, mais concernant une autre forme d'exécution du collier selon l'invention.

Dans ce dessin, la référence numérique 2 désigne le collier, 3 une barrette de renforcement, et 4 une pièce de soutien mentonnier.

Le collier 2 est constitué par une âme 2a en mousse synthétique à cellules fermées ayant, par exemple, une densité inférieure à 50kg/m3. Cette âme est disposée dans une enveloppe textile 2b, par exemple en coton. L'enveloppe est liée à la mousse par deux coutures extrêmes 5a, 5b assurant également la liaison de moyens d'accrochage complémentaires, tel q'un ruban à bouclettes 6a et un ruban à crochets 6b, et par une couture longitudinale traversante 7 s'étendant sur toute la longueur du collier et formant le bourrelet supérieur 8, qui est donc continu et permament.

La barrette de renfort 3 est réalisée dans une matière plastique semi-rigide et, par exemple, en polyéthylène haute densité, ayant une épaisseur comprise entre 0,8 et 1,2 mm. Elle présente la forme générale d'un « V » dont la concavité est tournée vers le haut. Cette barrette est liée au collier 2 par deux rivets encliquetables extrêmes 9 et par au moins un rivet encliquetable central 10, et de préférence deux rivets.

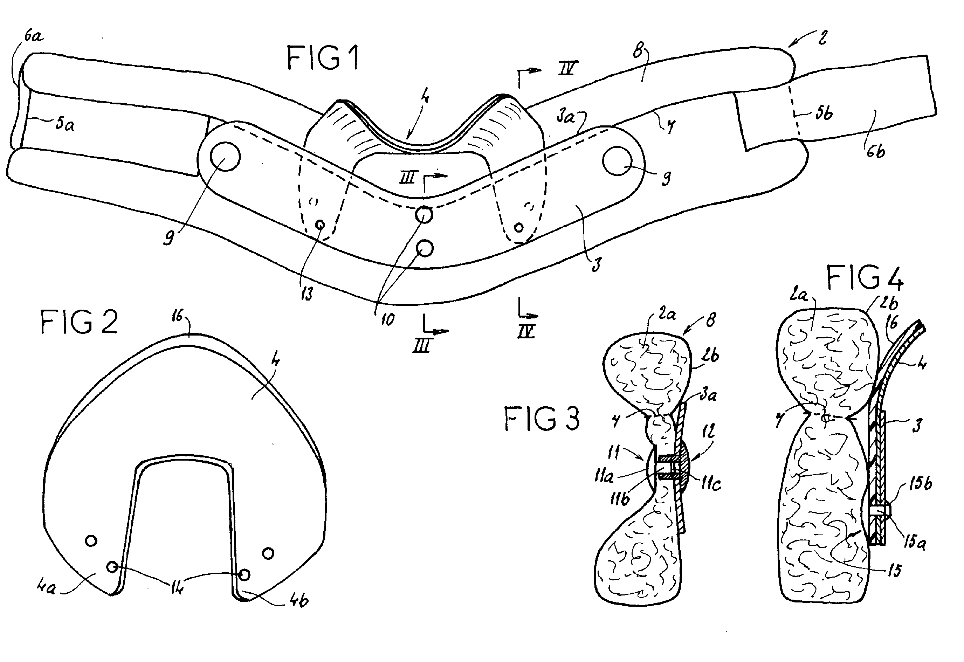

Comme montré plus en détails à la figure 3, dans une forme de réalisation, chaque rivet encliquetable est composé d'une partie mâle 11 avec une tête 11 a et une tige 11 b équipée d'un bourrelet 11 c, et d'une partie femelle 12, dont l'alésage axial est muni d'une gorge dans laquelle vient s'encliqueter le bourrelet 11c de la partie mâle 11.

La barrette 3 comporte, dans la forme d'exécution représentée aux figures 1 à 7, et de part et d'autre des rivets centraux 10, des perforations cylindriques 13 dont l'utilité sera précisée plus loin. La figure 1 montre que, lorsque la barrette 3 est mise en place sur le collier 2, son bord supérieur 3a vient en appui sur le bourrelet 8, de manière à renforcer ce bourrelet sans possibilité de passer au-dessous de lui en créant une zone résistante gênant le patient.

La pièce de soutien mentonnier 4 est réalisée en matière plastique semi-rigide et, par exemple, en polypropylène, ayant une épaisseur comprise entre 1 et 1,2 mm. Comme montré à la figure 2, elle présente la forme générale d'un «C » dont les deux branches 4a, 4b sont chacune traversées par au moins deux trous extrêmes 14. Lorsque cette pièce est mise en place sur le collier, l'écartement de ses deux branches 4a, 4b cintre sa partie centrale en lui donnant une concavité épousant sensiblement la forme du menton du patient. Les branches 4a, 4b sont fixées par des rivets encliquetables qui peuvent être identiques à ceux 9 et 10 ou être composés, comme montré à la figure 4, d'un unique rivet 15 dont la tige 15a est munie d'une extrémité élargie, excroissance ou bourrelet 15b, apte à s'encliqueter élastiquement sur la face antérieure de la barrette 3 après passage dans les trous 14 et 13 ménagés respectivement dans la pièce 4 et dans la barrette 3.

Selon la perforation 14 de la mentionnière 4 traversée par chaque rivet 15, cette mentonnière est plus ou moins haute par rapport au collier 2. Ce réglage est réalisé en fonction de la morphologie du patient.

La pièce de soutien 4 peut être associée à une nappe de confort 16 qui est alors fixée en même temps qu'elle par les rivets 15.

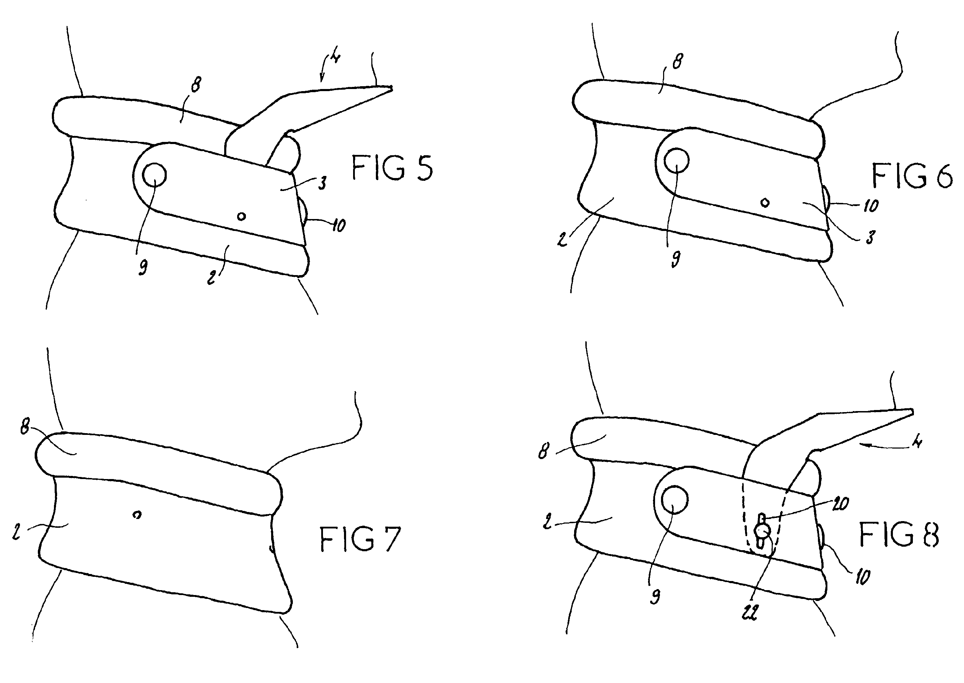

Comme montré à la figure 5, au début du traitement, le collier 2 est équipé de la barrette de renfort 3 et de la pièce 4 de soutien mentonnier. Dès que les muscles des vertèbres cervicales se sont raffermis, la pièce 4 peut être enlevée, par simple désencliquetage des rivets 15, sans affecter aucunement le soutien apporté par la pièce 3, comme montré à la figure 6. Dans une phase suivante, consécutive à un meilleur raffermissement des muscles, la pièce de soutien 3 peut, à son tour, être enlevée comme montré à la figure 7 en permettant au patient d'utiliser le même collier jusqu'à la fin du traitement.

La forme d'exécution représentée à la figure 8 se différencie de la précédente par le fait que les deux trous 13 ménagés dans la pièce de soutien 3 sont remplacés par des lumières verticales 20, tandis que les rivets 15 sont remplacés par un ensemble tige filetée-écrou 22.

Grâce à cela, il est possible, au début du traitement, d'ajuster la position verticale de la mentonnière 4 en faisant coulisser les ensembles 22 dans les lumières 20, puis de bloquer la mentonnière dans cette position par vissage.

Avec ce mode de liaison, le démontage de la mentonnière 4 nécessite de dévisser totalement les tiges filetées des écrous, ce qui nécessite une opération guère plus complexe que dans la forme d'exécution précédente.

Il ressort de ce qui précède que, grâce à sa structure, le collier cervical selon l'invention, assure un maintien évolutif et réglable au fur et à mesure de l'évolution du traitement, sans qu'il soit besoin de le remplacer, ce qui réduit le coût du traitement et améliore le confort du patient qui n'a pas à s'habituer à des colliers successifs.

## Revendications

1. Collier cervical à maintien réglable composé d'un collier (2) avec bourrelet supérieur(8) et dont les extrémités sont munies de moyens d'accrochage complémentaires (6a, 6b) permettant son maintien autour du cou, une barrette de renfort (3) et d'une pièce (4) de soutien du menton comprenant deux branches (4a, 4b), **caractérisé en ce que** le collier (2) est constitué par une âme (2a) en mousse à cellule ouverte, cette âme étant disposée dans une enveloppe textile (2b) à laquelle elle est liée par des coutures extrêmes (5a, 5b) et par une couture longitudinale traversante (7), ladite couture s'étendant sur toute la longueur du collier pour former le bourrelet supérieur (8), lequel est donc continu et permanent, tandis que la barrette (3) en matière plastique semi-rigide, ayant la forme générale d'un « V » et dont le bord supérieur prend appui sur le bourrelet (8), au-dessus de la couture longitudinale (7), est liée au collier (2) par deux moyens de liaison démontables extrêmes et par au moins un moyen de liaison démontable central et comportant, dans chacune de ses deux branches, au moins une perforation (13) pour des moyens de fixation démontables des extrémités libres des deux branches (4a, 4b) de la pièce (4) de soutien mentonnier amovible.

2. Collier selon la revendication 1, **caractérisé en ce que** les moyens de fixation démontables des deux branches (4a, 4b) de la pièce (4) de soutien mentonnier sur la barrette de renfort (3) comprennent des rivets encliquetables traversant les perforations, respectivement (13 et 14), ménagées dans ces deux pièces.

3. Collier selon la revendication 1, **caractérisé en ce que** chaque branche (4a, 4b) de la pièce (4) comporte plusieurs perforations (14) alignées, aptes chacune à être traversée par un unique rivet encliquetable (12), traversant également une perforation (13) de la barrette.

4. Collier selon la revendication 1, **caractérisé en ce que** les moyens de fixation démontables des deux branches (4a, 4b) de la pièce (4) de soutien mentonnier sur la barrette de renfort (3) sont montés coulissants avec possibilité de blocage dans deux lumières verticales (20) de réglage, réalisées dans la barrette (3) de renfort.

5. Collier selon la revendication 1, **caractérisé en ce que** la partie centrale de la barrette (3) est liée à l'âme (2a) du collier par deux rivets encliquetables (10) superposés traversant la barrette (3)de l'âme (4a).

6. Collier selon la revendication 1, **caractérisé en ce que** chaque rivet encliquetable (15) comprend, sur sa tige (15a), une tête élargie qui s'encliquète derrière l'élément rigide traversé.

7. Collier selon la revendication 1, **caractérisé en ce que** chaque rivet encliquetable (9, 10) est composé d'une partie mâle (11) et d'une partie femelle tubulaire (12) traversant les éléments à assembler et s'encliquetant après appui de leur tête sur lesdits éléments.

8. Collier selon l'ensemble des revendications 1 et 4, **caractérisé en ce que** chacune des branches (4a, 4b) de la pièce (4) de soutien mentonnier est fixée sur la barrette (3) par un ensemble tige filetée-écrou (22) dont la tige traverse la lumière (20) ménagée dans la barrette (3).

## Claims

1. Adjustable cervical collar, composed of a collar (2) which has an upper bead (8) and whose ends are equipped with complementary fastening means (6a, 6b) allowing it to be kept in place around the neck, of a reinforcement strip (3), and of a chin support piece (4) comprising two branches (4a, 4b), **characterized in that** the collar (2) is formed by a core (2a) of open-cell foam, this core being arranged in a textile envelope (2b) to which it is joined by end seams (5a, 5b) and by a longitudinal cross seam (7), said seam extending along the full length of the collar in order to form the upper bead (8), which is thus continuous and permanent, whereas the strip (3) made of semirigid plastic material, having the general shape of a V and with its upper edge bearing on the bead (8), above the longitudinal seam (7), is joined to the collar (2) by two detachable connecting means at the ends, and by at least one detachable connecting means at the centre, and comprises, in each of its two branches, at least one hole (13) for detachable fixation means of the free ends of the two branches (4a, 4b) of the removable chin support piece (4).

2. Collar according to Claim 1, **characterized in that** the means for detachable fixation of the two branches (4a, 4b) of the chin support piece (4) on the reinforcement strip (3) comprise snap-in rivets passing through the respective holes (13 and 14) formed in these two pieces.

3. Collar according to Claim 1, **characterized in that** each branch (4a, 4b) of the piece (4) comprises several aligned holes (14) which are each able to be traversed by a single snap-in rivet (12), which also passes through a hole (13) in the reinforcement strip.

4. Collar according to Claim 1, **characterized in that** the means for detachable fixation of the two branches (4a, 4b) of the chin support piece (4) on the reinforcement strip (3) are mounted so as to slide, with possible locking, in two vertical control slots (20) formed in the reinforcement strip (3).

5. Collar according to Claim 1, **characterized in that** the central part of the reinforcement strip (3) is joined to the core (2a) of the collar by two superposed snap-in rivets (10) that pass through the reinforcement strip (3) of the core (4a).

6. Collar according to Claim 1, **characterized in that** each snap-in rivet (15) comprises, on its stem (15a), a widened head which snaps in behind the rigid element it has passed through.

7. Collar according to Claim 1, **characterized in that** each snap-in rivet (9, 10) is composed of a male part (11) and of a tubular female part (12) passing through the elements to be joined together and snap-fitting after their head bears on said elements.

8. Collar according to Claims 1 and 4 together, **characterized in that** each of the branches (4a, 4b) of the chin support piece (4) is fixed on the reinforcement strip (3) by an assembly (22) of threaded rod and nut, of which the rod passes through the slot (20) formed in the reinforcement strip (3).

## Patentansprüche

1. Verstellbarer Halshaltekragen, mit einem Kragen (2) mit einem oberen Wulst (8), wobei die Enden des Kragens mit komplementären Ankoppelmitteln (6a, 6b) versehen sind, die sein Halten um einen Hals herum ermöglichen, mit einem Verstärkungsbügel (3) und einem Stützteil (4) für das Kinn, das zwei Schenkel (4a, 4b) aufweist, **dadurch gekennzeichnet, dass** der Kragen (2) aus einem Kern (2a) aus offenzelligem Schaum gebildet ist, wobei der Kern in einer Textilhülle (2b) angeordnet ist, mit der er über äußere Nähte (5a, 5b) und über eine längs verlaufende hindurchgehende Naht (7) verbunden ist, wobei sich die Naht über die gesamte Länge des Kragens erstreckt, um den oberen Wulst (8) zu bilden, der somit durchgehend und unveränderlich ist, während der Bügel (3) aus halbsteifem Kunststoff, der die allgemeine Form eines "V" aufweist und dessen oberer Rand sich auf dem Wulst (8) oberhalb der längs verlaufenden Naht (7) abstützt, über zwei äußere lösbare Verbindungsmittel und über zumindest ein mittleres lösbares Verbindungsmittel mit dem Kragen (2) verbunden ist und in jedem seiner zwei Schenkel zumindest eine Durchbohrung (13) für lösbare Befestigungsmittel für die freien Enden der zwei Schenkel (4a, 4b) des abnehmbaren Kinnstützteils (4) aufweist.

2. Halskragen nach Anspruch 1, **dadurch gekennzeichnet, dass** die lösbaren Befestigungsmittel zum Befestigen der zwei Schenkel (4a, 4b) des Kinnstützteils (4) an dem Verstärkungsbügel (3) einrastbare Nieten aufweisen, die durch die Durchbohrungen (13 bzw. 14) hindurchgehen, die in diesen zwei Teilen ausgebildet sind.

3. Halskragen nach Anspruch 1, **dadurch gekennzeichnet, dass** jeder Schenkel (4a, 4b) des Teils (4) mehrere in einer Linie ausgerichtete Durchbohrungen (14) aufweist, die jeweils geeignet sind, das durch sie eine einzelne einrastbare Niete (12) hindurchgeht, die auch durch eine Durchbohrung (13) des Bügels hindurchgeht.

4. Halskragen nach Anspruch 1, **dadurch gekennzeichnet, dass** die lösbaren Befestigungsmittel zum Befestigen der zwei Schenkel (4a, 4b) des Kinnstützteils (4) an dem Verstärkungsbügel (3) mit der Möglichkeit eines Feststellens verschiebbar in zwei vertikalen Langlöchern (20) zur Verstellung montiert sind, die in dem Verstärkungsbügel (3) ausgebildet sind.

5. Halskragen nach Anspruch 1, **dadurch gekennzeichnet, dass** der mittlere Abschnitt des Bügels (3) durch zwei übereinander angeordnete einrastbare Nieten (10) mit dem Kern (2a) des Kragens verbunden ist, die durch den Bügel (3) des Kerns (4a) hindurchgehen.

6. Halskragen nach Anspruch 1, **dadurch gekennzeichnet, dass** jede einrastbare Niete (15) an ihrem Schaft (15a) einen vergrößerten Kopf aufweist, der hinter dem steifen durchquerten Element einrastet.

7. Halskragen nach Anspruch 1, **dadurch gekennzeichnet, dass** jede einrastbare Niete (9, 10) aus einem männlichen Abschnitt (11) und einem rohrförmigen weiblichen Abschnitt (12) gebildet ist, die durch die zusammenzufügenden Elemente hindurchgehen und nach Abstützen ihres Kopfes auf diesen Elementen einrasten.

8. Halskragen nach der Kombination aus Anspruch 1 und 4, **dadurch gekennzeichnet, dass** jeder der Schenkel (4a, 4b) des Kinnstützteils (4) an dem Bügel (3) durch eine Anordnung aus einem mit Gewinde versehenen Schaft und einer Mutter (22) befestigt ist, von denen der Schalt durch die Öffnung (20) hindurchgeht, die in dem Bügel (3) ausgebildet ist.
